# EUROPÄISCHE PATENTANMELDUNG

(11) **EP 0 702 930 A1**
(43) Veröffentlichungstag der Anmeldung: **27.03.1996**
(21) Anmeldenummer: 95114861.8
(22) Anmeldetag: 21.09.1995
(51) Int. Cl.: A61B 5/00

(54) **System für die Erfassung und/oder Übertragung von Aktionsströmen**

(30) Priorität: 23.09.1994 DE 9415397 U
(71) Anmelder: Dipl.-Ing. Wilfried Sorg GmbH, D-88279 Amtzell-Korb (DE)
(72) Erfinder: Sorg, Wilfried, D-88279 Amtzell (DE)
(74) Vertreter: Patentanwälte Eisele, Otten & Roth

(57) **Zusammenfassung**

Es wird ein System für die Erfassung und/oder Übertragung von Aktionsströmen wie Gehirnströmen, EKG-Signale usw. von Patienten, Sportlern o. dgl., bestehend aus einem Sensor (1), einem akkumulatorbetriebenen Sender zur Aufnahme und Weitergabe des Sensorsignals (2), einer Empfangsstation (4) und separaten Einrichtungen zum Laden und Regenerieren des Akkumulators sowie zur Programmierung der Senderelektronik (3) vorgeschlagen, das eine einfache Handhabung und Wartung ermöglicht. Dies wird erfindungsgemäß dadurch erreicht, daß der Sender ein Sendergehäuse mit wenigstens zwei Kontaktstellen besitzt und die separaten Einrichtungen zum Laden, Regenerieren und Programmieren eine Einheit (3) bilden.

## Beschreibung

Die Erfindung betrifft ein System für die Erfassung und/oder Übertragung von Aktionsströmen wie Gehirnströme usw. von Patienten, Sportlern o. dgl. nach dem Oberbegriff des Anspruchs 1.

### Stand der Technik:

Systeme für die Erfassung und/oder Übertragung von Aktionsströmen wie Gehirnströme, Herzpuls usw. von Patienten, Sportlern o. dgl. sind aus der Medizintechnik und den Sportwissenschaften bekannt. Die Systeme dienen der Überwachung der oben genannten Körpersignale der Personen, die sich dabei frei bewegen können. Der Sensor und der akkumulatorbetriebene Sender zur Aufnahme und Weitergabe des Sensorsignals sind dazu an der Person angebracht. Beim Betrieb des Systems müssen die Akkumulatoren des Senders in regelmäßigen Abständen geladen und regeneriert werden. Unter Regenerieren versteht man einen Zyklus von definierten Entladungen und nachfolgenden Aufladungen zum Zwecke der Kapazitätssteigerung bei Akkumulatoren, die aufgrund ungünstiger Betriebsbedingungen eine Kapazitätseinbuße erfahren haben. Bei einer bekannten Ausführungsform werden die Akkus des Senders zum Laden und Regenerieren entnommen oder das Gerät wird an ein konventionelles Ladegerät angeschlossen. Die elektrische Verbindung erfolgt dabei z.B. durch einen Klinkenstecker. Darüberhinaus kann eine Umprogrammierung der Senderelektronik zur Anpassung an die Bedürfnisse der Anwendung notwendig sein. Im allgemeinen ist zur Umprogrammierung ein weiteres separates Gerät und ein zusätzlicher Steckkontakt am Sender erforderlich. Das gleichzeitige Laden, Regenerieren und Programmieren mehrerer Sender ist nur mit einer entsprechenden Anzahl von Lade- und Programmiergeräten durchführbar. Somit ist die Handhabung und Wartung dieser Systeme, was im wesentlichen das Laden, Regenerieren und Programmieren der Sender umfaßt, besonders für eine große Zahl von Sendern sehr aufwendig. Desweiteren besitzen die Sender aufgrund ihrer Steckkontakte eine Baugröße und ein Gewicht, wodurch die Bewegung des Patienten, Sportlers o. dgl. beschränkt sein kann. Ein aktiver Zugriff auf die Senderelektronik über die Ladekontakte zur Einsparung eines Steckkontaktes ist bei keiner der bekannten Ausführungsformen möglich.

### Aufgabe und Vorteile der Erfindung:

Der Erfindung liegt die Aufgabe zugrunde, die Handhabung und Wartung von Systemen für die Erfassung und/oder Übertragung von Aktionsströmen wie Gehirnströme, EKG-Signale usw. von Patienten, Sportlern o. dgl. zu verbessern.

Diese Aufgabe wird durch die Merkmale des Anspruchs 1 gelöst.

In den Unteransprüchen sind vorteilhafte und zweckmäßige Weiterbildungen des erfindungsgemäßen Systems angegeben.

Besonders vorteilhaft an der Erfindung ist es, daß das erfindungsgemäße System eine Einheit besitzt, mit der das Laden und Regenerieren der Akkumulatoren des Senders bzw. das Programmieren der Senderelektronik durchgeführt werden kann. Die separaten Einrichtungen zum Laden, Regenerieren sowie Programmieren werden somit in einem Gerät zusammengefaßt. Das Gerät besteht aus einem Gehäuse, das zumindest ein Aufnahmefach mit einem Einsteckplatz oder mehrere modular angeordnete Einsteckplätze zur Anordnung der Sender sowie ein Einstellorgan für die Programmierwertvorgabe besitzt. Jeder Einsteckplatz weist dem Sender angepaßte Führungs- und Kontaktelemente auf. Zur Kontaktierung des Senders sind mindestens zwei Kontakte, z.B. Federkontakte vorgesehen. Dadurch können die Sender in einfacher Weise eingesteckt und wieder entnommen werden, wobei im eingesteckten Zustand ein sicherer Kontakt mit der Lade-, Regenerier- und Programmiereinheit gewährleistet ist. Durch diese Art der Kontaktierung kann die Größe und das Gewicht des Senders soweit reduziert werden, daß es den Sportler, Patienten o. dgl. in seinen Bewegungen nicht beschränkt. Der Einsatz von lediglich zwei Kontaktstellen setzt eine Multifunktion der dazu kompatiblen Senderkontakte voraus. Der Sender muß demnach die von der Lade-, Regenerier- und Programmiereinheit vorgegebenen Zustände "Laden, Regenerieren oder Programmieren" durch seine Elektronik erkennen. Obgleich sich bei zwei Kontakten der Aufwand bei der Senderelektronik erhöht, bietet die Lösung einen Kostenvorteil gegenüber z.B. drei oder mehr Kontakten. Bei der Herstellung des Sendergehäuses aus Kunststoff müssen die Kontakte in die Spritzgußform eingelegt werden. Das Einlegen eines zusätzlichen (dritten) Kontaktes ist teurer als die geringfügig umfangreichere Elektronik.

Um die Einheit zum Laden, Regenerieren und Programmieren effektiv zu gestalten, ist es vorteilhaft, daß wenigstens einer der Steckplätze ausschließlich für die Regenerierung und die Programmierung des Senders genutzt werden kann. Die restlichen Einsteckplätze werden dann für das wesentlich häufiger vorkommende Laden eingesetzt.

Um eine möglichst einfache Handhabung und manuelle Einstellbarkeit der Einheit zu gewährleisten, ist es desweiteren günstig, die Umschaltung zwischen Regenerieren und Programmieren mit einem separaten Schalter sowie die Programmierwertvorgabe durch wenigstens einen Drehschalter o. dgl. zu realisieren.

Desweiteren ist es besonders vorteilhaft, daß der Sender ein hermetisch geschlossenes Sendergehäuse besitzt. Dadurch kann der Sender auch bei Patienten, Sportlern o. dgl. eingesetzt werden, die sich im Wasser oder einer sonst den Sender schädigenden Umgebung befinden.

Die Realisierung der Senderelektronik zur selbständigen Ladesteuerung des Akkumulators im hermetisch geschlossenen Sendergehäuse erfolgt vorteilhafterweise durch eine elektronische Spannungspegelweiche, einen Stromregler, einen "Single-Chip"-Mikroprozessor und Signalleitungen. Der Einsatz des Mikroprozessors erlaubt eine sehr variable Ladesteuerung und Programmierung des Senders. Die Spannungspegelweiche bewertet die Spannung, die an den Senderkontakten anliegt. Bei einem Spannungspegel von vorzugsweise 10 bis 14 Volt an den Senderkontakten erhält der Mikroprozessor über eine Signalleitung ein Ladesignal. Daraufhin gibt der Mikroprozessor an den Ladestromregler zur Ladung des Senderakkumulators eine in Zeitabschnitten (gepulste) Steuerspannung für den Ladestromregler aus.

In einer vorteilhaften Ausführung sind der Mikroprozessor, die Signalleitungen und die elektronische spannungspegelweiche des Senders so ausgelegt, daß der Mikroprozessor über die Senderkontakte und die serielle Schnittstelle eines Personal-Computers mit dem Personal-Computer kommunizieren kann. Damit besteht neben der Möglichkeit der Programmierung über die Lade-, Regenerier- und Programmiereinheit eine zusätzliche Möglichkeit auf die Senderelektronik Einfluß zu nehmen oder um Daten wie z.B. die Akkumulatorspannung des Senders auszulesen.

Im weiteren ist es günstig, die Spannungspegelweiche derart zu entwerfen, daß die Senderkontakte kurzschlußsicher sind. Dies ist insbesondere dann wichtig, wenn der Sender in einem leitenden Medium wie z.B. Wasser betrieben wird.

Um eine Überladung des im Sender integrierten Akkumulators zu verhindern, ist in der Lade-, Regenerier- und Programmiereinheit eine Stromüberwachung vorgesehen. Die Stromüberwachung kontrolliert den gepulsten Ladebetrieb. Dies ist von besonderem Interesse, wenn der Akkumulator des Senders tiefentladen ist. In diesem Zustand arbeitet der Mikroprozessor nicht, da er nicht ausreichend mit Energie versorgt wird. Eine Ladekontrolle von Seiten des Mikroprozessors ist damit nicht möglich. Wird nun an die Kontakte die Ladespannung gelegt, fließt zunächst ein konstanter nicht gepulster Ladestrom auf den Akkumulator. Die Stromüberwachung erkennt dies und bricht den Ladevorgang ab. Nach einer Wartezeit gibt die Stromüberwachung den Ladevorgang wieder frei und wiederum fließt ein konstanter Ladestrom auf den Akkumulator. Auf diese Weise wird der Akkumulator Schritt für Schritt so weit aufgeladen bis der Mikroprozessor wieder funktionsfähig ist und den gepulsten Ladevorgang, der von der Stromüberwachung erwünscht ist, übernimmt.

Letztlich ist es günstig, die Speicherkapazität des im Sender integrierten Akkumulators nach jedem Regeneriervorgang zu überprüfen. Dazu sind an der Regenerier-, Lade- und Programmiereinheit optische Anzeigeelemente wie z.B. Leuchtdioden, Kontrollampen o. dgl. vorgesehen.

### Zeichnungen:

Ein Ausführungsbeispiel der Erfindung ist in den Zeichnungen dargestellt. Es zeigen
- Fig. 1: ein System zur Erfassung und Übertragung eines EKG-Brustgurtsignals mit einer Lade-, Regenerier- und Programmiereinheit,
- Fig. 2: die Lade-, Regenerier- und Programmiereinheit in der Ansicht von oben,
- Fig. 3: die Einheit in der geschnittenen Seitenansicht,
- Fig. 4: das elektronische Blockschaltbild der Ladeeinheit und
- Fig. 5: das elektronische Blockschaltbild des Senders.

### Beschreibung des Ausführungsbeispiels:

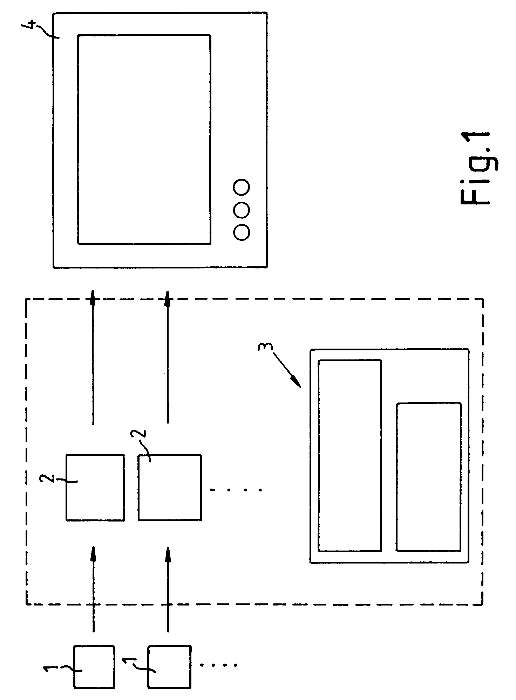

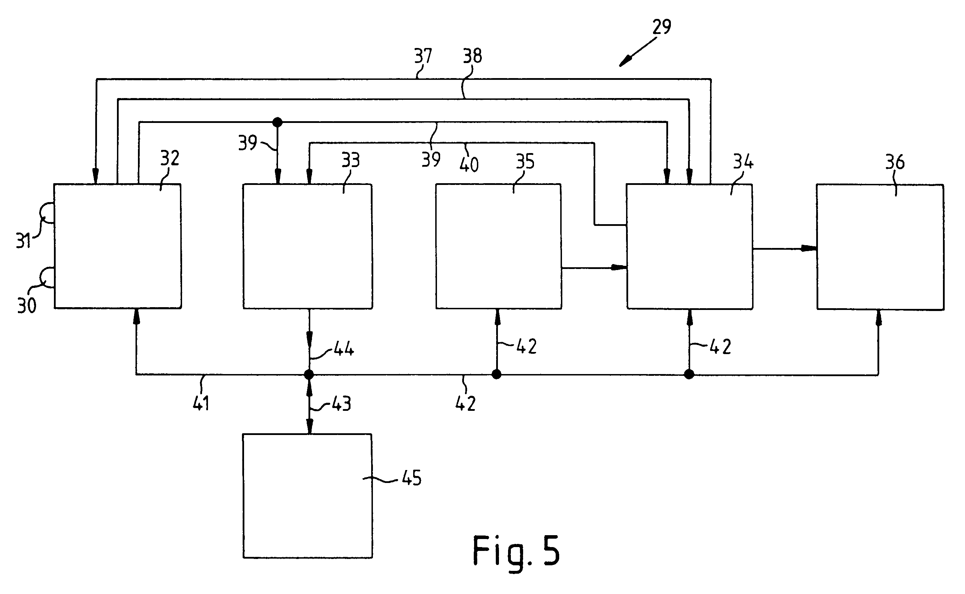

Das Ausführungsbeispiel aus Fig. 1 bis 5 besteht aus mehreren Brustgurtsensoren 1 (beispielsweise 5) und der gleichen Anzahl an Sendern 2, einer Einheit 3 zum Laden, Regenerieren und Programmieren der Sender und einer Basisstation 4. Die Lade-, Regenerier- und Programmiereinheit 3 besteht aus einem Gehäuse 5, das zwei Aufnahmefächer 6 und 7, und einen Schalter 8 zum Umschalten zwischen Regenerieren und Programmieren und zwei Drehschalter 9 und 10 für die Programmierwertvorgabe besitzt. Das Aufnahmefach 6 weist vier Sender-Einsteckplätze 11 bis 14 zum Laden der im Sendergehäuse integrierten Senderakkumulatoren auf. Das Aufnahmefach 7 besitzt drei Einsteckplätze 15 bis 17, wobei der dritte Einsteckplatz 17 zur Programmierung der Senderelektronik und zum Regenerieren des Senderakkumulators vorgesehen ist. Jeder Einsteckplatz setzt sich aus zwei Positionierbacken 18 und 19 mit zum Sender kompatiblen Führungsnasen 20, 21 und 22 zusammen. Zwischen den Führungsnasen 21 und 22 sitzen zur Kontaktierung des Senders zwei Federkontakte 23 und 24. Die Einsteckplätze 11 bis 17 sind in ihrer Bauweise vollkommen modular. Die Energiezuführung ins Gehäuse 5 erfolgt über die Durchführung 25.

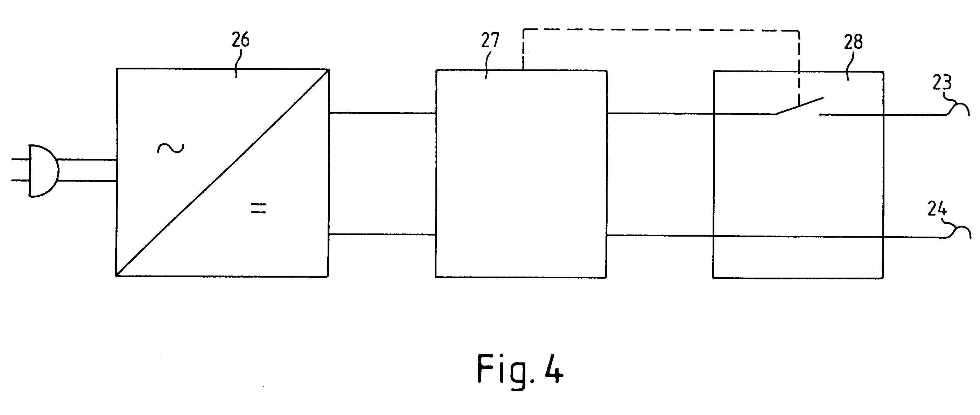

Eine Darstellung der elektronischen Baugruppen der Ladeeinheit wird in Fig. 4 gegeben. Die Ladeeinheit besteht aus einem Netzteil 26, einer Stromüberwachung der Pulsladung 27 und einem Stellglied 28, das gegebenenfalls bei ansprechender Stromüberwachung den Ladestromkreis unterbricht.

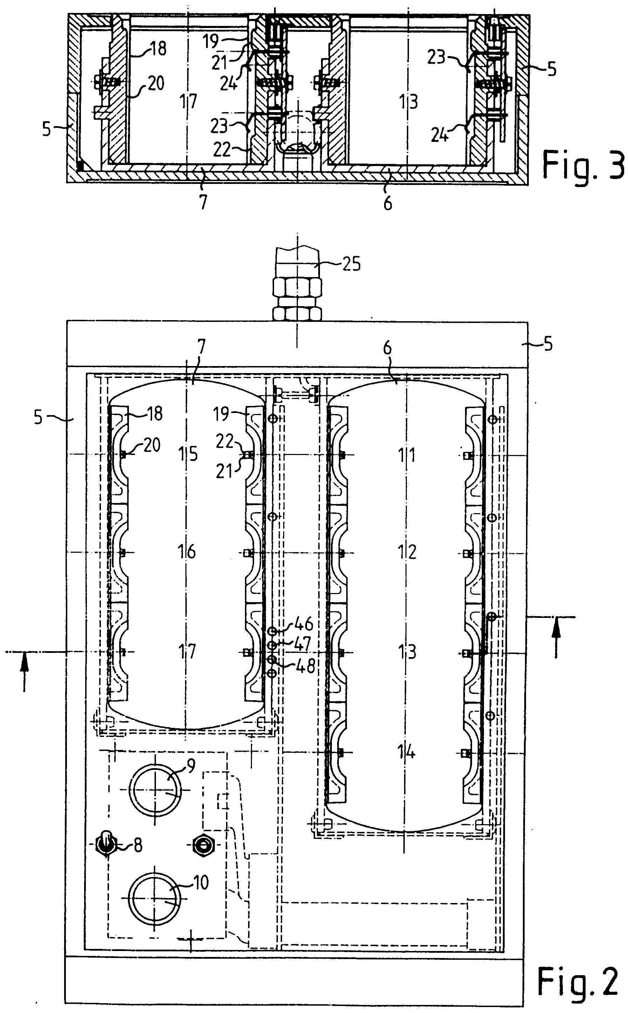

Das elektronische Blockschaltbild des Senders ist in Bild 5 dargestellt. Die Senderelektronik 29, die über die Senderkontakte 30, 31 angesprochen werden kann, setzt sich aus einer Spannungspegelweiche 32, einem Ladestromregler 33, einem "Single-Chip"-Mikroprozessor 34, einem Empfänger für das Brustgurtsensorsignal 35, einem HF-Modul 36 für die Übertragung des Brustgurtsensorsignals an die Basisstation 4, Signalleitungen 37 bis 44 und einem Akkumulator 45 zur Versorgung der Funktionsblöcke 35, 34 und 36 zusammen. Die Spannungspegelweiche 32 besteht im wesentlichen aus Zenerdioden. Liegt an den Kontakten 30, 31 eine Spannung zwischen 10 und 14 Volt an, gibt die Spannungspegelweiche 32 über die Signalleitung 38 ein Ladesignal an den Mikroprozessor 34. Daraufhin übermittelt der Mikroprozessor 34 über die Signalleitung 40 an den Ladestromregler 33 ein Signal, das zu einer gepulsten Ladung des Senderakkumulators 45 über die Signalleitungen 43, 44 führt. Während der Ladung des Akkumulators 45 erfolgt die Energieversorgung der Blöcke 33 und 34 aus dem Netzteil 26 über die Kontakte 23, 24, 30, 31, die Spannungspegelweiche 32 und die Signalleitung 39. Zur Regenerierung des Akkumulators ist ein Zyklus von definierten Endladungen und nachfolgenden Aufladungen notwendig. Die Endladung des Akkumulators 45 erfolgt über die Signalleitungen 43, 41, die Spannungspegelweiche 32 und die Kontakte 30, 31. Zur Sichtbarmachung der Speicherkapazität des Akkumulators 45 nach einem Regeneriervorgang sind am Gehäuse 5 der Lade-, Regenerier- und Programmiereinheit 3 neben dem Einsteckplatz 17 Leuchtdioden 46 bis 48 vorgesehen (vgl. Fig. 2). Die Kommunikation des Mikroprozessors 34 mit einem Personal-Computer über seine serielle Schnittstelle (nicht weiter gezeigt) erfolgt über die Signalleitung 37 der Spannungspegelweiche 32 und den Kontakten 30, 31.

Die Handhabung des Systems geschieht wie folgt:

Der Patient oder Sportler trägt bei der Pulserfassung im Herzbereich den Brustgurt-Sensor 1, der induktiv das EKG-Signal zum Sender 2 weitergibt. Der Sender 2 befindet sich an einer günstigen Stelle an der Kleidung des Patienten oder Sportlers und überträgt HF- moduliert das Signal zur Basisstation 4. Die Basisstation 4 kann gleichzeitig von mehreren Patienten oder Sportlern mit Brustgurtsensor 1 und Sender 2 bedient werden. Sender 2, die nicht gebraucht werden, können zum Laden, Regenerieren oder Programmieren in die Einheit 3 gesteckt werden. Die Regenerierung und Programmierung des Senders 2 wird im Einsteckplatz 17 vorgenommen, wobei die gewünschte Funktion durch den Schalter 8 definiert wird. Steht der Schalter 8 auf Programmieren, kann der Sportler über die Drehschalter 9 und 10 dem Sender 2 seine minimale und maximale Pulszahl einprogrammieren. Diese Grenzwerte werden vom Sender 2 an die Basisstation 4 übertragen.
- 1: Brustgurtsensor
- 2: Sender
- 3: Lade-, Regenerier- und Programmiereinheit
- 4: Basisstation
- 5: Gehäuse
- 6: Aufnahmefach
- 7: Aufnahmefach
- 8: Schalter
- 9: Drehschalter
- 10: Drehschalter
- 11: Einsteckplatz
- 12: Einsteckplatz
- 13: Einsteckplatz
- 14: Einsteckplatz
- 15: Einsteckplatz
- 16: Einsteckplatz
- 17: Einsteckplatz
- 18: Positionierbacken
- 19: Positionierbacken
- 20: Führungsnase
- 21: Führungsnase
- 22: Führungsnase
- 23: Federkontakt
- 24: Federkontakt
- 25: Durchführung
- 26: Netzteil
- 27: Stromüberwachung der Pulsladung
- 28: Stellglied
- 29: Senderelektronik
- 30: Senderkontakte
- 31: Senderkontakte
- 32: Spannungspegelweiche
- 33: Ladestromregler
- 34: "Single-Chip"-Mikroprozessor
- 35: Empfänger für das Brustgurtsensorsignal
- 36: HF-Modul
- 37: Signalleitung
- 38: Signalleitung
- 39: Signalleitung
- 40: Signalleitung
- 41: Signalleitung
- 42: Signalleitung
- 43: Signalleitung
- 44: Signalleitung
- 45: Akkumulator
- 46: Leuchtdiode
- 47: Leuchtdiode
- 48: Leuchtdiode

## Patentansprüche

1. System für die Erfassung und/oder Übertragung von Aktionsströmen wie Gehirnströme, EKG-Signale usw. von Patienten, Sportlern o. dgl., bestehend aus einem Sensor (1), einem akkumulatorbetriebenen Sender zur Aufnahme und Weitergabe des Sensorsignals (2), einer Empfangsstation (4) und separaten Einrichtungen zum Laden und Regenerieren des Akkumulators sowie zur Programmierung der
Senderelektronik (3), dadurch gekennzeichnet, daß der Sender ein Sendergehäuse mit wenigstens zwei Kontaktstellen besitzt und daß die separaten Einrichtungen zum Laden, Regenerieren und Programmieren eine Einheit (3) bilden, die aus einem Gehäuse (5) mit wenigstens einem Aufnahmefach (6, 7) und mit einem Einsteckplatz (11) oder mehreren modular angeordneten Einsteckplätzen zur Anordnung des Senders (2) und einem Einstellorgan (9, 10) für die Programmierwertvorgabe besteht, wobei jeder Einsteckplatz dem Sender angepaßte Führungs- und Kontaktelemente (18, 19, 23, 24) aufweist.

2. System für die Erfassung und/oder Übertragung von Aktionsströmen nach Anspruch 1, dadurch gekennzeichnet, daß die Einheit zum Laden, Regenerieren und Programmieren einen Einsteckplatz besitzt, der ausschließlich für die Regenerierung und Programmierung des Senders vorgesehen ist.

3. System für die Erfassung und/oder Übertragung von Aktionsströmen nach Anspruch 1, dadurch gekennzeichnet, daß die Einheit zum Laden, Regenerieren und Programmieren zur Umschaltung zwischen Regenerieren und Programmieren einen separaten Schalter (8) besitzt und das Einstellorgan für die Programmierwertvorgabe wenigstens einen Drehschalter (9, 10) o. dgl. umfaßt.

4. System für die Erfassung und/oder Übertragung von Aktionsströmen nach Anspruch 1, dadurch gekennzeichnet, daß der Sender (2) ein hermetisch geschlossenes Gehäuse besitzt.

5. System für die Erfassung und/oder Übertragung von Aktionsströmen nach Anspruch 1, dadurch gekennzeichnet, daß die Senderelektronik (29) zur selbständigen Ladesteuerung des Akkumulators (45) eine elektronische
Spannungspegelweiche (32), einen Ladestromregler (33), einen "Single Chip"-Mikroprozessor (34) und Signalleitungen (37 bis 44) umfaßt, wobei die Spannungspegelweiche (32) derart ausgebildet ist, daß diese bei einem Spannungspegel von vorzugsweise 10 bis 14 Volt an den Senderkontakten (30, 31) über eine Signalleitung (38) die Übertragung eines Ladesignals an den Mikroprozessor ermöglicht, und der Mikroprozessor (34) derart programmiert ist, daß dieser bei empfangenem Ladesignal eine in Zeitabschnitten definierte (gepulste) Ladung des Senderakkumulators (45) mittels des mit dem Mikroprozessor verbundenen Ladestromreglers (33) ermöglicht.

6. System für die Erfassung und/oder Übertragung von Aktionsströmen nach Anspruch 5, dadurch gekennzeichnet, daß der Mikroprozessor (34), die Signalleitungen und die elektronische spannungspegelweiche (32) des Senders (2) derart ausgelegt sind, daß über die Senderkontakte (30, 31) und die serielle Schnittstelle eines Personal-Computers eine Kommunizierbarkeit zwischen dem Mikroprozessor (34) und dem Personal-Computer gegeben ist.

7. System für die Erfassung und/oder Übertragung von Aktionsströmen nach Anspruch 5 oder 6, dadurch gekennzeichnet, daß zur Herstellung von kurschlußsicheren Senderkontakten (30, 31) eine elektronische Spannungspegelweiche (32) vorgesehen ist.

8. System für die Erfassung und/oder Übertragung von Aktionsströmen nach Anspruch 1, dadurch gekennzeichnet, daß die Einheit zum Laden, Regenerieren und Programmieren zur Sicherstellung des gepulsten Ladebetriebs des im Sender integrierten Akkumulators (45) eine Stromüberwachung (27) besitzt.

9. System für die Erfassung und/oder Übertragung von Aktionsströmen nach Anspruch 1, dadurch gekennzeichnet, daß zur Sichtbarmachung der Speicherkapazität des im Sender integrierten Akkumulators (45) nach jedem Regeneriervorgang optische Anzeigeelemente (46, 47, 48) vorgesehen sind.
